# EUROPEAN PATENT SPECIFICATION

(11) **EP 4 065 177 B1**
(45) Date of publication and mention of the grant of the patent: **25.06.2025**
(21) Application number: 21701020.6
(22) Date of filing: 17.01.2021
(51) Int. Cl.: A61L 2/07, A61L 2/24

(54) **STERILIZER**
STERILISATOR
STÉRILISATEUR

(30) Priority: 23.01.2020 US 202062964690 P
(43) Date of publication of application: 05.10.2022
(73) Proprietor: Tuttnauer Ltd., 9910101 Beit Shemesh (IL)
(72) Inventor: KINORY, Nir, Yehuda (IL)
(74) Representative: Caldon, Giuliano
(86) International application number: PCT/IB2021/050322
(87) International publication number: WO 2021/148920

(56) References cited:
- DE-A1- 19 715 583

## Description

This application claims priority to U.S. Provisional Patent Application Ser. No. 62,964,690 filed on January 23, 2020.

### FIELD OF THE INVENTION

The present invention relates to a sterilization device, a sterilization system and a method of sterilization.

### BACKGROUND OF THE INVENTION

Sterilization is carried out routinely in many industries and on diverse items, such as those related to the medical and food industries. Items to be sterilized can be placed in the sterilization chamber of a sterilization device and a sterilization cycle can be run using a suitable sterilization medium, such as for example steam and heat.

Sterilization cycles in steam sterilizers can be classified based on the performance of the cycle as Class B, Class N and Class S. These cycles differ in the way in which air is removed, the types of load they can sterilize and whether the items can be wrapped during sterilization. All three types remove air from the sterilization chamber, however the methods to attain this are different. Class N sterilizers use passive air removal from the sterilizer. Class S sterilizers use active air removal by for example steam pulsing and Class B sterilizers use active air removal by for example using a vacuum pump. Class N sterilization cycles are suitable for unwrapped solid items, whilst Class B is used for wrapped or unwrapped solid or hollow items. Class S sterilization cycles are suitable only for the types of load specified by the sterilizer manufacturer. Although Class B has the widest range of applications, it is typically more expensive and may take longer than a Class S sterilization cycle.

The guidelines regarding which type of sterilization cycle should be used for which type of item differ by country. In America, Class S sterilization is within the recommended guidelines for certain end uses and this type of sterilizer is typically used in for example tabletop sterilizers in dentistry and medical clinics. However, in view of the possibility that government guidelines may change, and/or a potential future need by a user for optimal sterilization on a wider variety of type of object, there may be a widespread need to replace Class N sterilizers with Class S or Class B sterilizers and Class S sterilizers with Class B sterilizers. Currently, a Class N or a Class S sterilizer may meet the needs of a user. The user may like to continue to use a machine that he is familiar with, as well as it typically being cheaper for him to run than a higher class sterilizer, such as a Class B sterilizer. As such the user may want to switch to a Class B sterilizer only when, or if it is necessary. Such a user may be averse to the expense of having to replace the Class N or Class S sterilizer at this future time with a costly Class B sterilizer.
DE 19715583 to Flecher Pierre Dr is directed to a medical steriliser and describes a single unit, wherein the three commercial CEN standard types 'B', 'N' and 'S' are combined and freely available to a user.

It would therefore be desirable to have a sterilizer which provides more than one class of sterilization. It would be useful to have a Class N or Class S sterilization device and wherein the same sterilizer can be adapted to provide Class B sterilization. It would further be advantageous to have a Class N or Class S sterilizer, which could be easily configured to provide Class B sterilization. It would be beneficial if the upgrade is significantly cheaper than replacing the Class N sterilizer or Class S sterilizer with a Class B sterilizer. It would be further environmentally desirable to provide an upgradable sterilizer, obviating the need to discard a redundant Class N or Class S sterilizer. The present invention provides such a system, device and method of use thereof.

### SUMMARY

The invention is set out in the appended set of claims.

The invention may have several aspects. One aspect is a sterilization device according to claim 1. The sterilization device includes a sterilization chamber for accommodating at least one item for sterilization. The sterilization device includes a power means for powering the device. The sterilization device includes a means for providing a sterilization medium for sterilizing the at least one item. The sterilization device includes a plurality of sterilization cycle programs. The plurality of sterilization cycle programs includes at least one S class sterilization cycle program and at least one B class sterilization cycle program. The at least one S class sterilization programs may be for sterilization of at least one item as defined by the manufacturer of the sterilization device. The at least one B class sterilization programs may be for sterilization of items for which Class S may be sufficient, in addition to items for which Class S is not adequate for sterilization, such as but not limited to at least one of a solid product, a porous product, a lumen device, a single-wrapped product, a multiple-layer product and a non-wrapped product. According to the invention, in a first state the sterilization device provides sterilization using the at least one S class sterilization cycle program and the at least one B class sterilization cycle program is locked and unavailable for use by a user. The sterilization device includes a second state wherein the sterilization device provides the at least one B Class sterilization cycle program unlocked and available for use by a user. The sterilization medium is steam for steam sterilization. The sterilization chamber may include at least one inlet for steam. The sterilization chamber may include at least one valve configured for connection to a vacuum system.

According to the invention, the at least one locked B class sterilization cycle program is configured to be unlocked with a code. The code may be provided separately from the sterilization device. According to the invention, the device includes a keyboard for inputting the or the code code may be inputted remotely.

In various embodiments of the sterilization device, the device includes at least one air removal system. The air removal system is configured to provide air removal from the sterilization chamber. The at least one air removal system may feature at least one of a steam pulsing system and a vacuum system. The sterilization chamber may include at least one inlet for at least one of steam and water. The sterilization chamber may include at least one outlet for at least one of air and steam. In an embodiment wherein the air removal system features a steam pulsing system, the steam pulsing system may be connected to the at least one inlet for steam and/or water of the sterilization chamber. The steam pulsing system may include water, which may be stored in a water tank, a water pump and a water heater. In an embodiment wherein the air removal system features a vacuum system, the vacuum system may be connected to the at least one valve of the sterilization chamber.

In various embodiments of the sterilization device, when the device is in a first state, the at least one program for providing B class sterilization is locked and the at least one air removal system is configured to provide air removal from the sterilization chamber sufficient for S class sterilization. The device may provide only S class sterilization.

In various embodiments the device is configured to a second state wherein the at least one program for B class sterilization cycle is unlocked. The at least one air removal system is configured to provide air removal sufficient for producing B class sterilization. The device may provide S class sterilization or B class sterilization. The device may provide only B class sterilization.

According to the invention, the sterilization device further includes at least one controller for controlling the sterilization cycles of the sterilization device. The sterilization device may further include at least one of a means for measuring pressure, a means for measuring temperature and a means for measuring time. The sterilization device may further feature at least one of a means for controlling pressure, a means for controlling temperature and a means for controlling time. The sterilization device may include at least one control for controlling the steam pulsing system. The sterilization device may include at least one control for controlling the vacuum system.

In various embodiments of the sterilization device, which are not part of the claimed invention, the plurality of sterilization cycle programs may further include at least one N class sterilization cycle. At least one of the at least one S class sterilization cycle program and the at least one B class sterilization cycle program may be in a locked state. In a first state the sterilization device may provide sterilization using the at least one N class sterilization cycle program and the at least one S class sterilization cycle program is locked and unavailable for use by a user and the at least one B class sterilization cycle program is locked and unavailable for use by a user. In a second state, the at least one S class sterilization cycle program may be unlocked and available for use by a user and the sterilization device may provide sterilization using either the at least one N class sterilization cycle program or the at least one S class sterilization cycle program and the at least one B class sterilization cycle program is locked and unavailable for use by a user. In a third state the sterilization device may provide sterilization using either the at least one N class sterilization cycle program or the at least one S class sterilization cycle program or the at least one B class sterilization cycle program. In a fourth state the at last one B class sterilization cycle program may be unlocked and available for use by a user and the sterilization device may provide sterilization using either the at least one N class sterilization cycle program or the at least one B class sterilization cycle program and the at least one S class sterilization cycle program is locked and unavailable for use by a user.

A further aspect is a sterilization system. The sterilization system includes a sterilization device and a code for unlocking at least one B class sterilization cycle of the sterilization device. The sterilization device includes a sterilization chamber for accommodating at least one item for sterilization. The sterilization device includes a means for powering the device. The sterilization device includes a means for providing a sterilization medium for sterilizing the at least one item. The sterilization device includes a plurality of sterilization cycle programs. The plurality of sterilization cycle programs includes at least one S class sterilization cycle program and at least one B class sterilization cycle program. The at least one B class sterilization cycle program is in a locked state. The sterilization device provides S class sterilization and is adaptable to unlock and provide B class sterilization.

In various embodiments of the sterilization system, the sterilization device includes at least one controller for controlling the at least one sterilization cycle of the sterilization device. The sterilization device may include at least one of a steam pulsing system and a vacuum system to provide air removal from the sterilization chamber.

A still further aspect, which is not part of the claimed invention, is a sterilization system featuring a sterilization device. The device includes a sterilization chamber for accommodating at least one item for sterilization, a means for powering the device, a means for providing a sterilization medium for sterilizing the at least one item and a plurality of sterilization cycle programs. The plurality of sterilization cycle programs may include at least one N class sterilization cycle program, at least one S class sterilization cycle program in a locked state and at least one B class sterilization cycle program in a locked state. The sterilization device may provide N class sterilization and is adaptable to unlock and provide S class sterilization and B class sterilization. The sterilization system may include a code for unlocking the at least one locked S class sterilization cycle and a code for unlocking the at least one locked B class sterilization cycle. The sterilization device may include a passive steam air removal system and at least one of a steam pulsing system and a vacuum system to provide air removal from the sterilization chamber.

An aspect is a method of sterilization. The method features providing a sterilization device of the present invention. The sterilization device is in a first state, wherein the at least one B class sterilization cycle program is locked. The method includes placing at least one item in the sterilization chamber and running at least one S class sterilization cycle for sterilizing the at least one item.

An aspect is an additional method of sterilization. The method features providing a sterilization device of the present invention. The method includes placing at least one item in the sterilization chamber and selecting an S class sterilization cycle or a B class sterilization cycle. The method further includes running the selected class sterilization cycle for sterilizing the at least one item.

A still further aspect is a method of converting a sterilization device for providing S class sterilization into a sterilization device for providing B class sterilization. The method includes providing a sterilization device of the present invention. The sterilization device is in a first state, wherein the at least one B class sterilization cycle program is locked. The method includes inputting a code for unlocking the at least one B class sterilization cycle program for providing the sterilization device with at least one available B class sterilization cycle program and for converting the sterilization device from a first state wherein the at least one B class sterilization cycle is locked into a second state wherein the at least one B class sterilization cycle program is unlocked.

Another aspect is a further method of sterilization. The method includes providing a sterilization device of the present invention. The sterilization device is in a first state, wherein the at least one B class sterilization cycle program is locked. The method includes unlocking the at least one B class sterilization cycle program to provide at least one available B class sterilization cycle program. Unlocking may feature inputting a code. The method includes placing at least one item in the sterilization chamber and running at least one B class sterilization cycle for sterilizing the at least one item. The method may include selecting a B class sterilization cycle.

An aspect is a method of sterilization using a sterilization device which features at least one N class sterilization cycle program, at least one S class sterilization cycle program and at least one B class sterilization cycle program. The method includes providing a sterilization device of the present invention, wherein the sterilization device incudes a plurality of sterilization cycle programs, which feature at least one N class sterilization cycle program, at least one S class sterilization cycle program and at least one B class sterilization cycle program. The method includes placing at least one item in the sterilization chamber. The method includes selecting one of an N class sterilization cycle, an S class sterilization cycle or a B class sterilization cycle and running the selected class sterilization cycle to sterilize the at least one item. According to the invention, the at least one B class sterilization cycle program is in a locked state. The method may include unlocking the at least one B class sterilization cycle program to provide at least one available B class sterilization cycle program. The method may include running at least one B class sterilization cycle for sterilizing the at least one item.

### BRIEF DESCRIPTION OF THE DRAWINGS

The various features of the invention will best be appreciated by simultaneous reference to the description which follows and the accompanying drawings, which are not drawn to scale and in which:
FIG. 1 shows a schematic view of an exemplary sterilizer according to an aspect of the present invention;
FIGs 2a and 2b show schematic views of at least part of an exemplary system for providing sterilization conditions to a sterilizer of the present invention;
FIGs 3a and 3b show schematic views of an exemplary sterilizer according to an aspect of the present invention;
FIG. 4 shows a schematic view of exemplary programs of an exemplary sterilizer according to an aspect of the present invention;
FIGs 5a and 5b shows schematic views of exemplary programs of an exemplary sterilizer with an unlocked Class B sterilization cycle program according to an aspect of the present invention;
FIGs 6a-6e show schematic views of an exemplary sterilizer system according to an aspect of the present invention;
FIG. 7 shows a flow chart of an exemplary method of converting an exemplary Class S convertible sterilizer to a Class B sterilizer according to an aspect of the present invention;
FIG. 8 shows a flow chart of an exemplary method of converting an exemplary Class N convertible sterilizer to a Class B sterilizer according to an aspect of the present invention;
FIG. 9 shows a flow chart of an exemplary method of use of an exemplary sterilizer according to an aspect of the present invention;
FIG. 10 shows a flow chart of an exemplary method of use of an exemplary sterilizer according to an aspect of the present invention; and
FIG. 11 shows a flow chart of an exemplary method of use of an exemplary sterilizer according to an aspect of the present invention.

### DETAILED DESCRIPTION

In one aspect the present invention is of a sterilization device. The sterilization device is configured to provide at least one S class sterilization cycle and at least one B class sterilization cycle. The sterilization device provides S Class sterilization and may be adaptable to provide B class sterilization. In an additional aspect the present invention is of a sterilization device which is configured to provide at least one N class sterilization cycle, at least one S class sterilization cycle and at least one B class sterilization cycle. In a further aspect, the present invention provides a sterilization system featuring a sterilization device and a key for unlocking B class sterilization cycles of the sterilization device. In a further aspect, the present invention provides a method of converting a sterilization device to a B Class sterilization device. Furthermore, the present invention provides a method of sterilization.

The device and system of the present invention have many advantages. The invention ensures that a user is not limited to use of only one class of sterilization from a single sterilizer. The invention provides a user with the option of converting a same S Type sterilizer to a B sterilizer at a future date. The invention provides a user with the option of converting a same N Type sterilizer to an S Type sterilizer and/or a B Type sterilizer. The invention also provides a way of reducing waste caused by throwing out of redundant S Type sterilizers and redundant N Type sterilizers. The invention may also enable a user to choose between S Type and B Type sterilization with a single sterilizer. The invention may also facilitate a user having the choice of using an N Type, S Type or B Type sterilization with a single sterilizer.

As used herein the terms 'Class' and 'Type' may be used interchangeably.

As used herein the terms 'locked' and 'locked state' may include, but are not limited to a state where a sterilization program or cycle is configured so that it cannot be used by a user without being unlocked in a suitable way. The unlocking may be by a suitable key.

As used herein the term 'available' may include, but is not limited to a state where the cycle is configured such that it can be used and run by a user. The term includes the state where the cycle is not locked.

As used herein the terms 'adapting' and 'adapted' may include, but are not limited to upgrading and reconfiguring of the sterilizer. The terms may include any suitable method of adapting, such as but not limited to using physical and/or software means.

As used herein the term 'distribution system' may include, but is not limited to any suitable means for providing a material from a first location to a second location. The term includes providing a material from one part of a system to another part of a system.

As used herein the term 'sterilization chamber' may include, but is not limited to a compartment where sterilization may be carried out.

As used herein the term 'connection' may include, but is not limited to direct and indirect attachment.

As used herein the term 'inlet' may include, but is not limited to a means of entry. The term may include any suitable means of entry, such as, but not limited to vents, ducts, pipes, flues and openings.

As used herein the term 'outlet' may include, but is not limited to a means of exit. The term may include an opening or passage configured for letting something out. The term may include vents, pipes, ducts and exits for expelling something.

As used herein the term 'channel' may include, but is not limited to a passage or a structure, which provides a passage or pathway for a material, such as a gas, vapor or liquid to flow along. The term may include a channel made from a material resistant to the material it is carrying. The term may include a channel for a separated passage of a material.

As used herein the term 'sterilization device' may include, but is not limited to a sterilizer, which features a sterilization chamber. The sterilizer may provide the sterilization chamber with sterilization conditions, materials, and other means for facilitating a sterilization process and sterilization of items in the sterilization chamber.

As used herein the term 'sterilization' may include, but is not limited to elimination, killing, removal or deactivation of biological agents, such as, but not limited to microorganisms, pathogens, bacteria, viruses, fungi, spore forms and prions from a specified region. Sterilization may be performed using a sterilization process and may run for longer than is required to provide a sterility assurance level. The term may include a process providing a sterility assurance level of at least 10⁻⁶.

As used herein the term 'disinfection' may include, but is not limited to a process to destroy microorganisms on a specified region. The term includes a method, which is less effective than sterilization at killing microorganisms and may not kill all microorganisms on a specified area, such as resistant bacterial spores.

As used herein the term 'sterilization cycle' may include, but is not limited to at least one stage of a sterilization procedure. The sterilization cycle may include all the stages of the sterilization procedure or only at least one stage.

As used herein the terms 'a' and 'an' may mean 'one' or 'more than one'.

As used herein the terms 'comprising', 'including', 'containing', 'featuring', 'having' and any forms of the terms thereof are inclusive and open ended and do not exclude additional, elements or methods steps, which are not recited.

The principles and operation of a system and device, such as a sterilizer, as well as methods of use thereof according to the present invention may be better understood with reference to the figures. The figures show non-limiting aspects of the present invention.

### The Sterilization Device

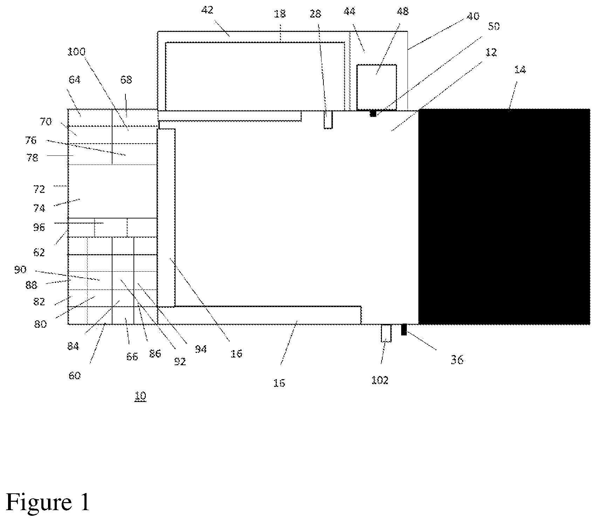

Figure 1 shows a schematic view of an exemplary sterilizer 10 according to an aspect of the present invention. The sterilizer 10 is configured as a sterilizer for providing S Class sterilization. Non-limiting examples of suitable sterilizers 10 for providing S Class sterilization include steam sterilizers and tabletop autoclaves. The sterilizer 10 is also configured as a sterilizer for providing B Class sterilization. The sterilizer may be a two class sterilizer providing S Class and B Class sterilization cycles.

In one aspect, the sterilizer features a plurality of states. In a first state, the sterilizer is provided wherein the B Class sterilization cycles are unavailable for use by a user and the user can only use the sterilizer in this first state for S Class sterilization. In this first state, the B Class sterilization cycle programs are in a dormant or locked state. The sterilizer 10 may be adapted to a second state wherein the B Class sterilization cycles are available for use.

The sterilization device 10 features a sterilization chamber 12. The sterilization chamber 12 may be any suitable sterilization chamber and may be constructed from any suitable material, such as, but not limited to stainless steel or polycarbonate. The sterilization chamber 12 may be of any suitable size and shape for sterilizing at least one item. Figure 1 shows an example of a square shaped sterilization chamber 12, however this is not intended to be limiting. The sterilization chamber 12 may be a single chamber or may include multiple chambers or multiple sub compartments. The sterilization chamber 12 may include any suitable door 14, which can be closed during a sterilization cycle. The door may include any opening and closing mechanism. The sterilizer 10 may include any suitable shelving or racks for placement of items to be sterilized.

The sterilizer 10 includes at least one system for providing sterilization conditions to the sterilization chamber 12. The at least one system for providing sterilization conditions to the sterilization chamber may include a heating device 16 for providing heat to the sterilization chamber 12. The heating device may be any suitable heating device. One non-limiting example of a suitable heating device is an electrical jacket heater for heating the sterilization chamber. The heating device 16 may be configured so that it surrounds the sterilization chamber 12 or may be positioned in any other suitable way so that it provides uniform heat to the sterilization chamber. The heating device may provide heat of a temperature greater than about 100 °C. In some embodiments, the heating device may provide a temperature of greater than about 120 °C.

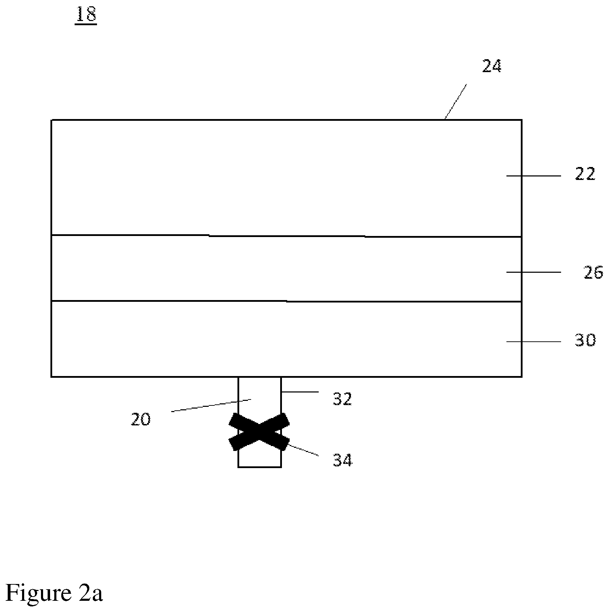

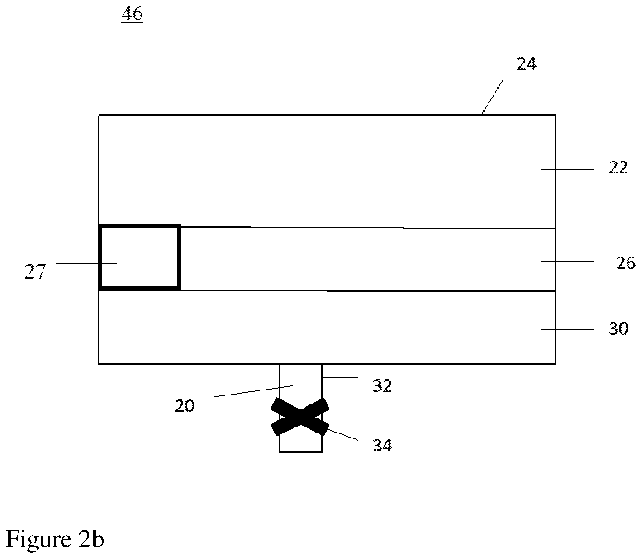

The at least one system for providing sterilization conditions to the sterilization chamber may include a means 18 for providing steam 20 to the sterilization chamber 12. The means 18 for providing steam 20 to the sterilization chamber may be positioned externally from the sterilization chamber 12. As shown schematically in Figure 2a, the means 18 for providing steam 20 to the sterilization chamber 12 may include a steam supply 20 or a source of suitable water 22 for conversion to steam 20. The water 22 may be stored in a reservoir 24. In some embodiments, the sterilizer 10 may be connectable to an external water source, such as a water tap. A suitable water pump 26 may pump the water. The means 18 for providing steam 20 to the sterilization chamber 12 may be suitably connected to at least one steam inlet 28 of the sterilization chamber 12 to provide steam 20 into the chamber 12. In one non-limiting embodiment, the steam 20 may be produced by a steam generator 30, such as but not limited to an electrical pipe water heating system 30. The resulting steam 20 may be provided to the sterilization chamber by a steam distribution channel 32, such as, but not limited to a tubing 32 via a steam valve, which may be upstream 34 and which may be connected to the inlet port 28 of the sterilization chamber 12. The water pump 26 may facilitate providing the steam to the sterilization chamber 12 for sterilization of the contents of the sterilization chamber 12. The water pump 26 may provide water to the steam generator 30 and the steam may flow from the steam generator 30 to the sterilization chamber 12. The water pump 26 may be controlled by a pump control 27 to provide control of steam pulsing. As such, the means 18 for providing steam 20 to the sterilization chamber may be configured as a steam pulsing system 46, which may feature the water pump 26 and the water heating system as shown in Figure 2b.

In an alternative example, which is not shown in Figure 1, the means 18 for providing steam 20 to the sterilization chamber 12 may be positioned in the sterilization chamber 12. In such an example, the means 18 for providing steam 20 may not need a water pump 26 and may produce and provide the steam by a water source and a heating system.

As shown schematically in Figure 1, the at least one system for providing sterilization conditions to the sterilization chamber may include means for active air removal 40 from the sterilization chamber 12. The sterilization chamber 12 may include at least one outlet 36 for releasing air and/or steam. In one embodiment shown in Figure 1, the sterilizer 10 of the present invention has a means for sufficient active air removal for Class S sterilization 42 and the sterilizer 10 has an additional means for sufficient active air removal for Class B sterilization 44. The means for sufficient air removal for Class S sterilization 42 may include a means for providing steam pulsing 46 to the sterilization chamber 12 as described hereinabove and in Figure 2b and may include the means 18 for providing steam 20 to the sterilization chamber configured as a steam pulsing system 46. The means for sufficient air removal for Class B sterilization 44 may include a vacuum system 48, such as but not limited to a vacuum pump 48 connected in a suitable way to the sterilization chamber 12. In one non-limiting embodiment, the vacuum system 48, such as the vacuum pump 48 is connected to the sterilization chamber 12 with a valve 50. The vacuum system 48 may include any device or component for providing a sufficient vacuum. An additional non-limiting example of a suitable vacuum system 48 is a water pump.

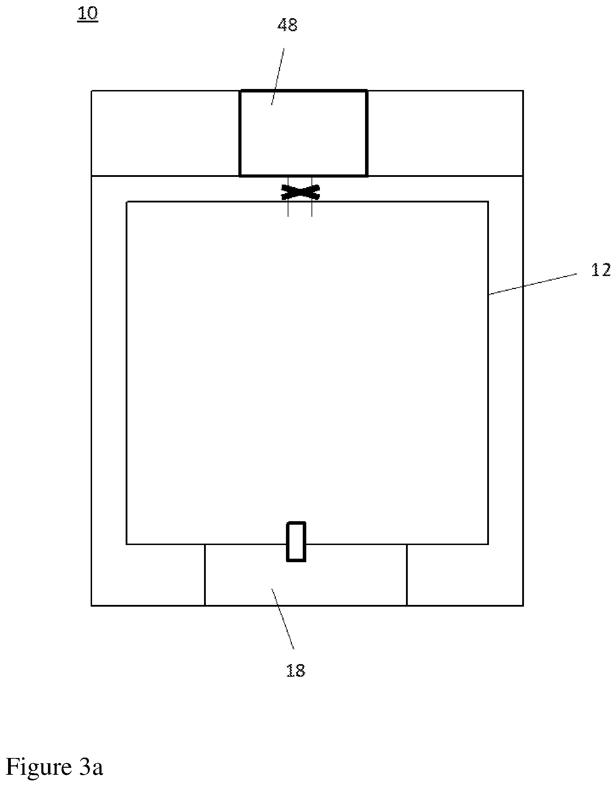

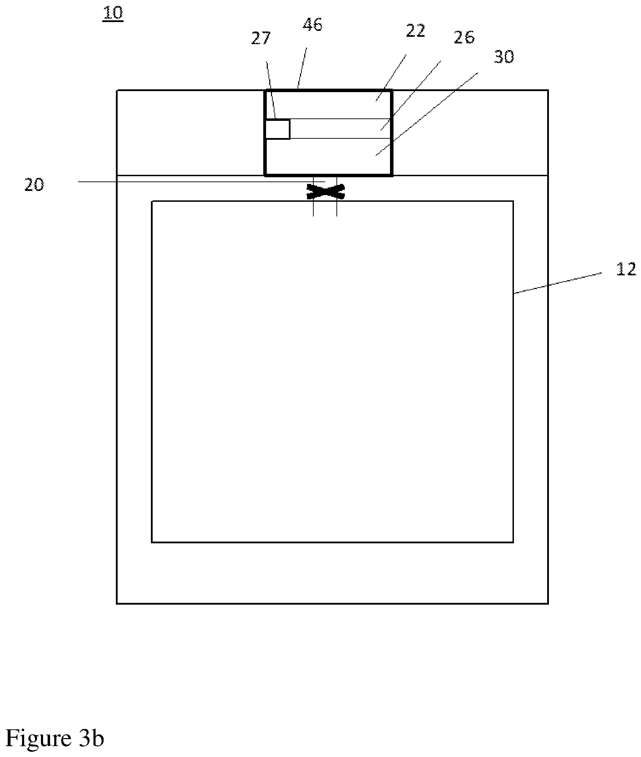

In an alternative embodiment, shown schematically in Figure 3a and Figure 3b, the sterilizer has a means which can be operated to provide sufficient air removal for both Class S and Class B sterilization. The same means may be operated differently with different conditions depending on the Class of sterilization to be achieved. In one non-limiting example shown in Figure 3a, the means may be a vacuum system 48, such as a vacuum pump 48, which can be operated using conditions for providing Class S sterilization 42 and which can be operated using different parameters for Class B sterilization 44. In such an example, the sterilizer device includes means 18 for providing steam 20 to the sterilization chamber, which in one non-limiting example may be a steam pulsing system 46. However, in this example the function of the steam pulsing system and its operation, is to provide steam for sterilization without the need for the steam pulsing system to be controlled to remove a certain amount of air to result in the desired Class of sterilization. In an alternative non-limiting example, shown schematically in Figure 3b, the means to provide sufficient air removal for both Class S and Class B sterilization may be a steam pulser 46, which can be operated using conditions for providing Class S sterilization and which can be operated using different parameters for Class B sterilization. In a further non-limiting example, the means may include a combination of a vacuum system 48, such as a vacuum pump 48 and a steam pulser 46 which can be operated using respective conditions for Class S 42 or Class B 44 sterilization.

As shown schematically in Figure 1, the sterilizer 10 includes a control 60. The figure shows one non-limiting arrangement of the features of the control. The device may include any suitable arrangement and order of the control components. The control may include a switch 62 to start and stop the sterilization device. The control may include a suitable keyboard 64. The control may include power controlling means 66. The control may include a clock 68 for measuring time. The control may include a timer 70 for setting the time of the sterilization cycle. The control may include a screen 72 and a display 74.

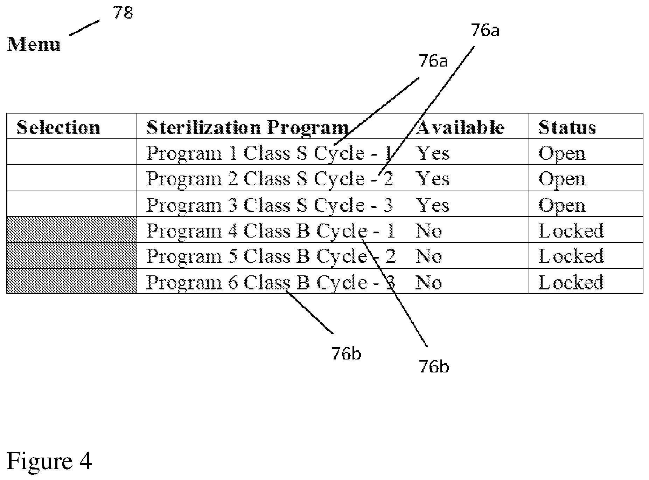

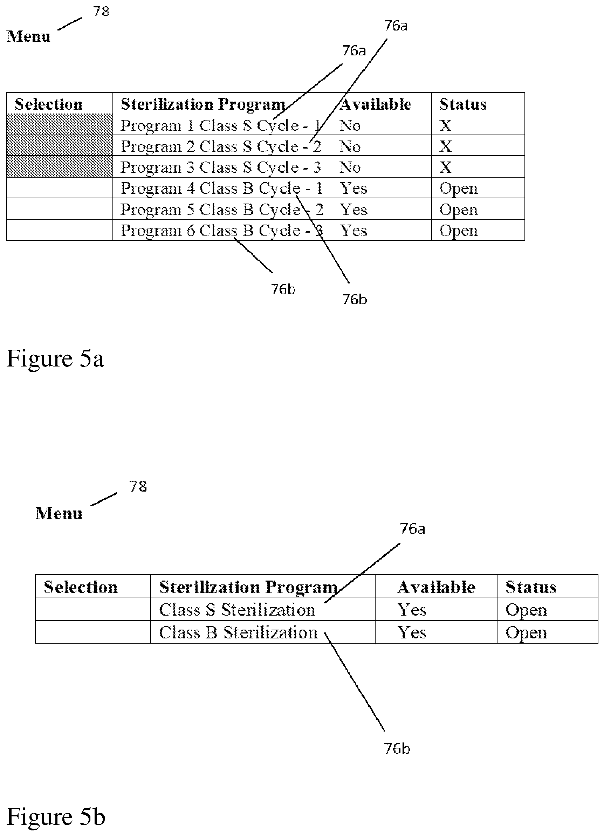

The control 60 includes different programs 76 of sterilization cycle. The programs may be provided as part of a menu 78. The programs 76 may be preset and/or may be programmable. Figure 4 shows a schematic view of exemplary programs of an exemplary sterilizer according to an aspect of the present invention. The control 60 includes at least one program 76a for a S Class sterilization cycle 42. The at least one program for S Class sterilization may include any suitable number of programs 76a. The control 60 includes at least one program 76b for a B Class sterilization cycle 44. The at least one program 76b for B Class sterilization may include any suitable number of programs 76b. In one non-limiting example, the sterilization device 10 may include at least two B Class sterilization cycle programs 76b. Non-limiting examples of B Class sterilization cycle programs 76b may include a 121 °C B Class cycle and a 134 °C B Class cycle. As previously described, in a first state the at least one program 76b for a B Class sterilization cycle may be unavailable to a user. The sterilizer may include the components for providing B Class sterilization, but in this state the B Class sterilization cycle program 76b may be locked in any suitable way to prevent a user from accessing or using a B Class sterilization cycle. Typically, a user may purchase the sterilizer device of the present invention with the device configured to run S Class sterilization and with the B Class sterilization programs in a locked, unusable state. In such a state, the user can only run Class S sterilization. The sterilizer device may be adaptable to provide the option of a B Class sterilization program to the user. The sterilizer may be adapted, such that the locked B Class sterilization cycle program may be opened with a suitable key resulting in the sterilizer in a second state wherein a B Class sterilization cycle can be run by a user. When a user does not have a key to unlock the B Class sterilization cycles, the B Class sterilization will not be available for use to the user. According to the invention, a suitable key is a code. The code is inputted using the keyboard or the code may be inputted remotely. The code may be given to a user in any suitable way, such as, but not limited to by electronic mail, postal delivery, phone, verbally, over the WWW (IoT) network and combinations thereof. The code may be a unique code, which is configured to be different for each device. The code may be any suitable code, such as, but not limited to a QR code, a barcode, a 2D code, a sequence of a suitable number of numbers or, letters, or symbols or combinations thereof. Opening of the locked B Class sterilization cycle and selection of a B Class sterilization cycle may activate the means and parameters sufficient for air removal for Class B sterilization as described above. In one embodiment, unlocking of the B Class sterilization cycles may circumvent the sterilizer S Class sterilization system to only provide a user with options of B Class sterilization cycles 76b as shown schematically in Figure 5a. In an alternative embodiment, unlocking of the B Class sterilization cycles may provide a user with options of operating either a S Class sterilization cycle 76a or a B Class sterilization cycle 76b as shown schematically in Figure 5b. In such an embodiment, a user may select which type of sterilization, S Class or B Class to run on an item to be sterilized in the sterilizer device. In a further alternative embodiment, the sterilizer may be provided with both S Class and B Class sterilization classes in an unlocked state and with no need to unlock the sterilizer to provide B Class sterilization. In such an embodiment a user may select either a S Class sterilization cycle 76a or a B Class sterilization cycle 76b as shown schematically in Figure 5b.

In some embodiments, the locked sterilization class may be unlocked once and may then remain unlocked. In one embodiment, the at least one B Class sterilization cycles 76b may be relockable. In one non-limiting example, the unlocking of the at least one B Class sterilization cycle 76b may be for a specific time duration after which the B Class sterilization cycles relock 76b. Alternatively, the at least one B Class sterilization cycle 76b may be locked remotely. Such a relockable sterilizer may be suitable for a subscription payment scheme for use of the B Class Sterilization cycles. In one embodiment, the at least one B Class sterilization cycle once unlocked may be made unavailable. Unavailability of B Class sterilization may result from a selection choice in a sterilization cycle menu, such as but not limited to selecting an S Class sterilization menu.

The controller 60 may include means for controlling the sterilization cycles of the sterilization device. The controller may include a heating control 80 for controlling the temperature provided to the chamber. The device may include at least one thermometer 82 for measuring the temperature. The controller may include a control 84 for controlling the production of steam. The controller may include a means for controlling pulsing of steam 86 provided to the incubation chamber. The controller may control the water pump to control pulsing of steam, which may be by controlling the pump control 27. The controller may include a means for controlling pressure 88. The controller may include a pressure measuring device 90. The controller may include a means for controlling the time of a sterilization cycle 92. The controller may include a means for opening and closing steam inlets 94 for allowing steam into the incubation chamber. The controller may include a means for controlling operation and parameters of a vacuum system, such as a vacuum pump 96. The controller may include means for closing and opening steam and air outlets of the sterilization chamber.

The sterilizer includes a power supply 100 and electrical connections for powering the sterilization device 10 and its components. The power supply 100 may be any suitable power supply that supplies power. The sterilizer may include a safety valve 102.

The sterilizer may be constructed from materials suitable for Class S sterilization conditions and suitable for Class B sterilization conditions.

In one non-limiting embodiment, which is not part of the claimed invention, the sterilizer may be configured to provide three classes of sterilization cycle, Class N, Class S and Class B. In such an embodiment, the three class sterilizer may have four states. In a first state, sterilization cycles/s of Class N may be available to a user. The sterilization cycles of Class S and Class B may be locked in any suitable way as described herein for Class B, such as with a lock, which can be opened with a suitable key. In one non-limiting example a different key may unlock each of the locked classes. In a second state, Class S sterilization cycle/s may be unlocked providing a user with unlocked and available sterilization cycles of Class N and Class S. In a third state, Class S and Class B sterilization cycles may be unlocked to provide a user with unlocked and available sterilization cycles of Class N, Class S and Class B. In a fourth state, Class B sterilization cycles may be unlocked providing a user with unlocked and available Class N and Class B sterilization cycles. In one non-limiting embodiment, the three class sterilizer may be provided with Class N, Class S and Class B sterilization cycles, which are available to a user on selection of the desired class of sterilization.

The three class sterilizer may feature substantially the same components as the two class S and B sterilizer 10 described hereinabove. To avoid repetition the components of the sterilizer 10 which provide Class S and Class B sterilization will not be described again. In the three class sterilizer, Class N sterilization may be provided by gravity displacement of air using steam. Referring to Figure 1, the means 18 for providing steam 20 to the sterilization chamber 12, which is available in the previously described two class S and B sterilizer can provide steam to the sterilization chamber for passive air removal facilitating Class N sterilization conditions. In a first state, the sterilization device may be configured to only provide passive air removal for Class N sterilization and the conditions and components for providing Class S and Class B are not activated. They can be locked or they can be open and available only on selection of that Class and the resulting reconfiguration of the device, such as unlocking or activating the conditions relating to the selected class of sterilization cycle. Unlocking Class S sterilization cycles may feature opening and making available to the sterilization device air removal by steam pulsing or by a vacuum system, such as a vacuum pump configured with parameters for Class S sterilization as previously described. Unlocking Class B sterilization cycles may feature opening and making available to the sterilization device air removal by steam pulsing or a vacuum system, such as a vacuum pump configured with parameters for Class B sterilization as previously described. The sterilizer may be constructed from materials suitable for Class N sterilization conditions, Class S sterilization conditions and Class B sterilization conditions.

The sterilizer of the present invention may be suitable for a variety of sterilization applications. Non limiting examples include sterilization of instruments used in medical clinics and all types of dental clinic.

### Sterilization System

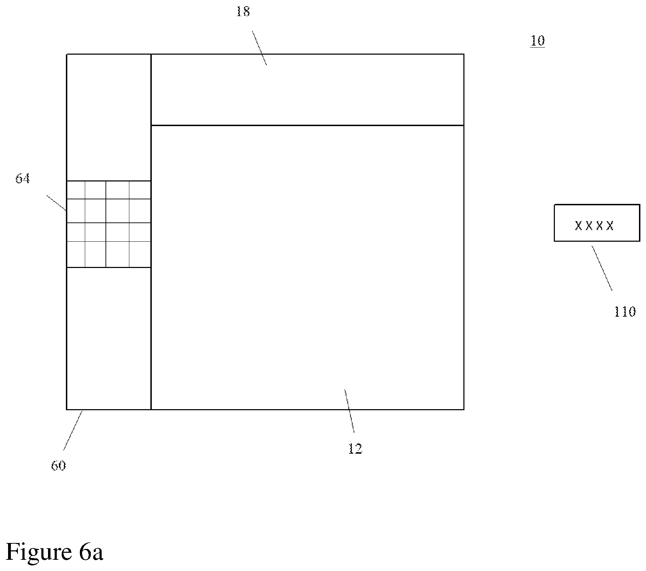

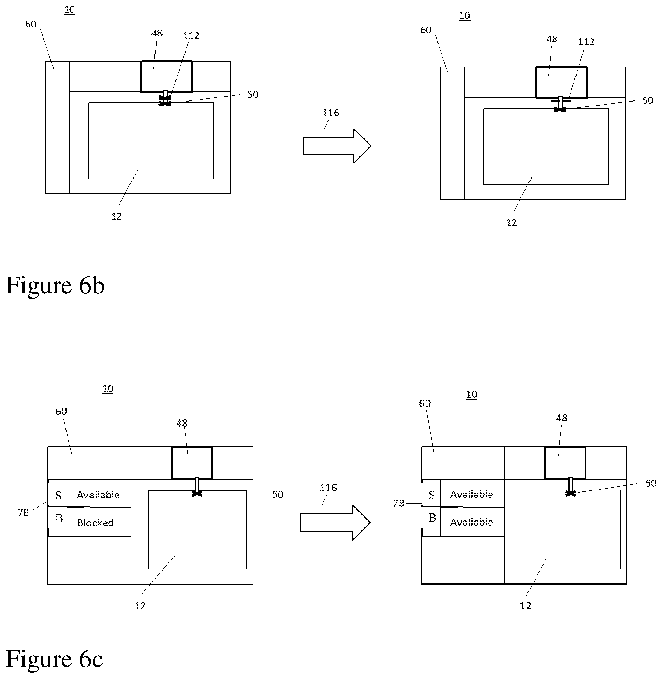

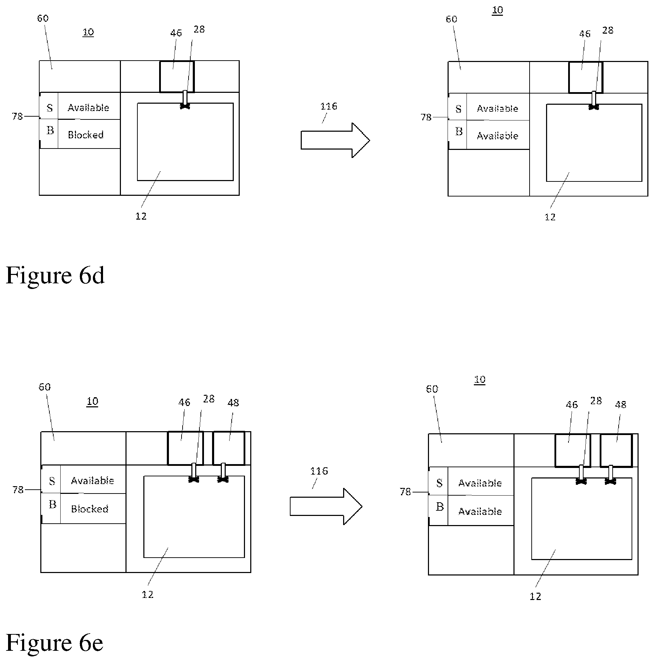

The present invention provides a sterilization system as shown schematically in Figure 6a. The system features a sterilization device 10 as described hereinabove and in Figures 1-4. The device may be a device configured to provide Class S sterilization and Class B sterilization, wherein in a first state the Class B sterilization cycles are locked and unavailable for use. The sterilization system includes a key 110 for unlocking at least one Class B sterilization cycle of the sterilizer 10. In one non-limiting example the key 110 may be a code. The code may be inputted by a user using the sterilizer keyboard 64 or by any suitable method. The code may include software commands to open Class B sterilization cycles. In one non-limiting example, the code may include software commands to lock Class S sterilization cycles. In an alternative non-limiting embodiment, the key may be a physical key. Use of the key with the sterilizer device may facilitate physical connection to the sterilization chamber of any necessary opening or activation of means for active air removal sufficient for Class B sterilization. The physical key may also cause disconnection from the sterilization chamber of the means for active air removal sufficient for Class S sterilization. Figure 6b shows schematically a non-limiting example of a sterilizer of the present invention including a vacuum system, such as, but not limited to a vacuum pump to provide Class B sterilization. The vacuum system, such as the vacuum pump 48 may include at least one valve 112, which may be provided closed and which may be configured to prevent air removal of the sterilization chamber by the vacuum system, such as the vacuum pump. The vacuum system, such as the vacuum pump may be connected to at least one additional valve 50 to provide control of the air evacuation. Adapting 116 the sterilizer by use of the key 110 may cause opening of the valve 112 on the vacuum system, such as the vacuum pump to allow sufficient air removal from the sterilization chamber and control by valve 50 to provide Class B sterilization. In one embodiment, the key may cause opening and/or closing of any suitable inlet or outlet for facilitating unblocked access of the air removal system to the sterilization chamber for sufficient air removal to provide the target class of sterilization on selection of that sterilization class. In an alternative embodiment as shown schematically in Figure 6c, a sterilizer may include a vacuum system, such as a vacuum pump 48 configured to provide Class B sterilization, wherein the vacuum system, such as a vacuum pump does not include the at least one valve 112 as described for Figure 6b. The sterilizer may be adapted to unblock Class B sterilization cycles by employing the code 110. The code 110 may facilitate use of Class B sterilization cycles by providing commands of a suitable sequence, order and parameters for sufficient air removal by the vacuum system, such as the vacuum pump to provide Class B sterilization. In one embodiment as shown schematically in Figure 6d, the sterilizer may include a steam pulser 46, which can be configured to provide both Class S and Class B sterilization. The sterilizer with blocked Class B sterilization cycles may be adapted 116 to facilitate Class B sterilization by employing the code 110, which provides commands, including commands to the steam pulser 46 of a suitable sequence, order and parameters for sufficient air removal to provide Class B sterilization. In some embodiments as shown schematically in Figure 6e, a sterilizer with blocked Class B sterilization cycles may include both a vacuum system, such as a vacuum pump 48 and a steam pulser 46 to provide both Class S and Class B sterilization. The sterilizer may be adapted 116 to unblock the Class B sterilization cycles by use of the code 110 which provides commands to facilitate Class B sterilization. Commands may include commands to the relevant air removal system such as to at least one of the steam pulser and the vacuum system, such as the vacuum pump of a suitable sequence, order and parameters for sufficient air removal to provide Class B sterilization.

In one non-limiting embodiment, which is not part of the claimed invention, the system may include a sterilization device configured to provide Class N, Class S and Class B sterilization, wherein at least one of the Class S and Class B sterilization cycles are locked. The system may include two keys 110, a first key for unlocking Class S sterilization cycles and a second key for unlocking Class B sterilization cycles. The key for unlocking Class B sterilization cycles may work as described for the key for unlocking Class B sterilization cycles in a Class S and Class B sterilizer. Use of the key for unlocking Class S sterilization cycles may facilitate physical connection and/or commands to the sterilization chamber of any necessary opening or activation of means or conditions for active air removal sufficient for Class S sterilization.

### Method of Converting a Sterilizer to a B Class Sterilizer

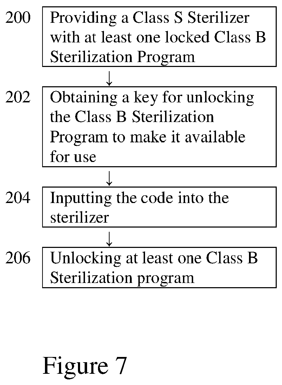

The present invention provides a method of converting a convertible Class S sterilizer of the present invention to a Class B sterilizer. Figure 7 shows a flow chart of an exemplary method of converting an exemplary convertible Class S sterilizer to a Class B sterilizer according to an aspect of the present invention. The method features providing a Class S sterilizer of the present invention 200, which is upgradeable to a sterilizer providing Class B sterilization. The sterilizer includes at least one Class B sterilization cycle program which is locked and which in its Class B locked state cannot be used by a user for Class B sterilization. A user can only use the upgradeable Class S sterilizer in this Class B locked state for a Class S sterilization cycle. The method includes a user obtaining a key for unlocking at least one Class B program 202. The key may be obtained by purchasing it from a suitable source, such as, but not limited to the manufacturer of the sterilizer. The key may be any suitable key, which may include a code. The method features the user inputting the code into the sterilizer device 204. Inputting may be by any suitable way, such as, but not limited to typing the code using a keyboard of the sterilizer. The code may work in any suitable way, such as by software commands to unlock the Class B sterilization cycles. Unlocking may include at least one of activating Class B sterilization cycle programs and physical connection of means for providing conditions for Class B sterilization to the sterilization chamber 206, as described hereinabove. Once the Class B sterilization cycle is unlocked, the sterilizer is configured to provide Class B sterilization, which is available for a user to run a Class B sterilization cycle.

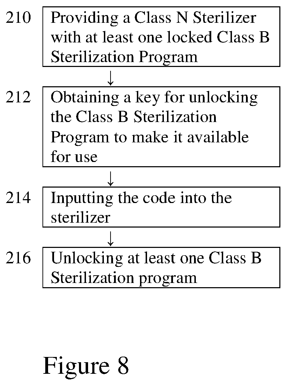

Figure 8 shows schematically a flow chart of an exemplary method, which is not part of the claimed invention, of converting an exemplary sterilizer providing N Class sterilization to a sterilizer providing B Class sterilization. The method features providing a Class N sterilizer of the present invention 210, which is upgradeable to a sterilizer providing Class B sterilization. The sterilizer includes at least one Class B sterilization cycle program which is locked and which in its Class B locked state cannot be used by a user for Class B sterilization. A user can only use the upgradeable Class N sterilizer in this Class B locked state for a Class N sterilization cycle. The method includes a user obtaining a key for unlocking the at least one Class B program 212. In an embodiment wherein the key is a code, the method features the code being inputted into the sterilizer 214. Inputting the code may be by any suitable method. Inputting the code facilitates unlocking at least one Class B Sterilization program so that it is available for use by a user 216.

### Method of Sterilization

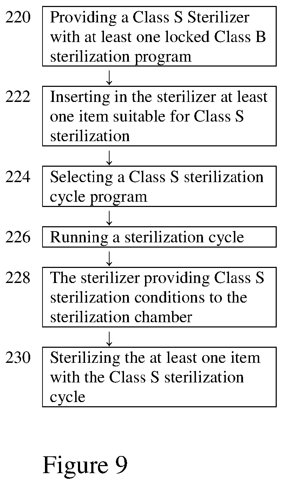

The present invention provides a method of sterilizing at least one item as shown schematically in the flow chart of Figure 9. The method includes providing a sterilizer of the present invention, the sterilizer providing Class S sterilization cycles, which are available for use by a user and the sterilizer including at least one Class B sterilization cycle, which is locked and unavailable for use by a user 220. The method includes a user inserting at least one item to be sterilized in the sterilizer 222. The at least one item to be sterilized is an item specified for suitability to be sterilized by a Class S sterilization cycle. The user selects a Class S sterilization program 224. The user runs the sterilization cycle 226 and the sterilizer works to provide the sterilization chamber with the Class S sterilization conditions 228. The Class S sterilization cycle sterilizes the at least one item 230. The order of the steps is not meant to be limiting and any suitable order may be used.

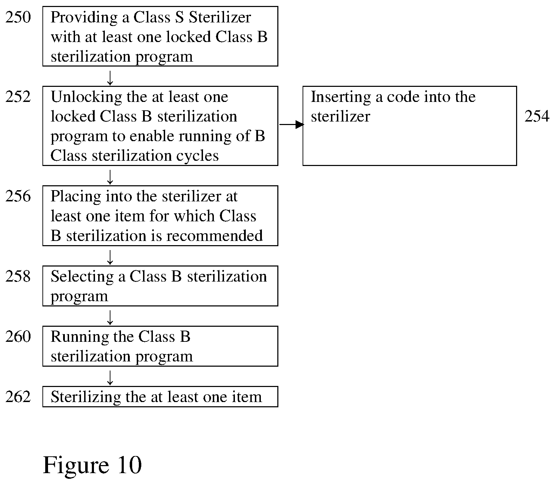

**In** an alternative embodiment, the present invention provides a method of sterilizing at least one item as shown schematically in the flow chart of Figure 10. The method includes providing a sterilizer of the present invention, the sterilizer providing Class S sterilization cycles, which are available for use by a user and the sterilizer including at least one Class B sterilization cycle, which is locked and unavailable for use by a user 250. A user may unlock the sterilizer of the present invention 252 for enabling Class B sterilization as described hereinabove and in Figure 7. Unlocking may be achieved by inputting a code into the sterilizer 254, which may be done using the sterilizer keyboard. A user may insert at least one item to be sterilized into the sterilization chamber 256. The at least one item may be an item for which the Class S sterilization cycle was not recommended as being sufficient for sterilization of that item and wherein Class B sterilization is needed for sterilization of that item. The user selects a Class B sterilization cycle from the program options 258. The user runs the Class B sterilization program 260. The sterilizer provides the incubation chamber with the Class B sterilization conditions and the at least one item is sterilized under these conditions 262. The order of the steps is not meant to be limiting and any suitable order may be used.

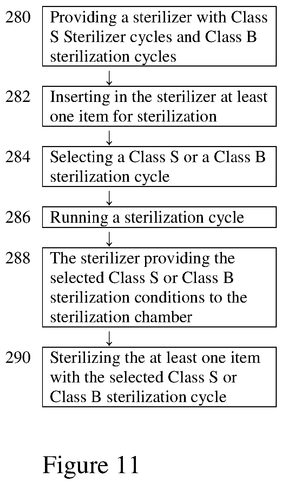

In a further embodiment, the present invention provides a method of sterilizing at least one item as shown schematically in the flow chart of Figure 11. The method includes providing a sterilizer of the present invention, the sterilizer providing at least one Class S sterilization cycle, which is available for use by a user and the sterilizer providing at least one Class B sterilization cycle, which is available for use by a user 280. The method includes a user inserting at least one item to be sterilized 282 in the sterilizer. The user selects a Class S or Class B sterilization cycle 284. Selection may depend on the item to be sterilized and which class of sterilization is needed for sterilization of that item. In one non-limiting example, the sterilization device may automatically select which class of sterilization is needed. The user runs the sterilization cycle 286 and the sterilizer works to provide the incubation chamber with the sterilization conditions of the selected class of sterilization 288. The selected sterilization class sterilizes the at least one item 290. The order of the steps is not meant to be limiting and any suitable order may be used.

Reference is made to the following example, which together with the above descriptions illustrates the invention in a non-limiting fashion.

### Example 1

A dentist purchases a sterilizer of the present invention which provides S Class sterilization and is upgradeable to provide B Class sterilization. She uses the standard mode of the sterilizer for sterilizing dental equipment specified as being suitable for S Class sterilization. The dentist expands her practice and some equipment of her associate cannot be sterilized by S Class sterilization. She does not want to buy a B Class sterilizer. Instead, the dentist buys the code for converting the S Class sterilizer of the present invention to a sterilizer that can provide B Class sterilization. She inputs the code and the B Class sterilization programs are unlocked and available to use. She has upgraded the sterilizer, which can now sufficiently sterilize all the equipment of her clinic.

One skilled in the art can appreciate from the foregoing description that the broad systems, devices and techniques of the aspects of the present invention can be implemented in a variety of forms. Therefore, while the aspects of this invention have been described in connection with particular examples thereof, the true scope of the aspects of the invention should not be so limited since other modifications will become apparent to the skilled practitioner upon a study of the specification, and following claims.

## Claims

1. A sterilization device (10) comprising:
a sterilization chamber (12) for accommodating at least one item for sterilization;
a power means (100) for powering the device;
a means for providing a sterilization medium (18) for sterilizing the at least one item;
wherein the sterilization medium is steam for steam sterilization;
and
a plurality of sterilization cycle programs (76) comprising:
at least one S class sterilization cycle program (76a); and
at least one B class sterilization cycle program (76b);
the sterilization device (10) comprises a controller (60) for controlling the sterilization cycles of the sterilization device (10)
and **characterised in that**,
in a first state the sterilization device (10) provides sterilization using the at least one S class sterilization cycle program (76a) and the at least one B class sterilization cycle program (76b) is locked and unavailable for use by a user;
in a second state the sterilization device (10) provides the at least one B Class sterilization cycle program unlocked and available for use by a user;
wherein the at least one locked B class sterilization cycle program is configured to be unlocked with a code; and
wherein the sterilization device (10) comprises a keyboard for inputting the code into the controller (60) or the code can be inputted remotely.

2. The sterilization device of claim 1, wherein the device (10) comprises at least one air removal system (40) and wherein the air removal system (40) is configured to provide air removal from the sterilization chamber (12) and wherein the at least one air removal system (40) comprises a steam pulsing system (46) comprising water, a water pump and a water heater and wherein the sterilization chamber comprises:
at least one inlet (28) for at least one of steam and water; and
at least one outlet (36) for at least one of air and steam; and
wherein the steam pulsing system is connected to the at least one inlet (28) of the sterilization chamber (12).

3. The sterilization device of claim 1, wherein the device (10) comprises at least one air removal system (40) and wherein the air removal system is configured to provide air removal from the sterilization chamber (12) and wherein the at least one air removal system (40) comprises a vacuum system (48) and wherein the sterilization chamber (12) comprises at least one valve (50) and the vacuum system (48) is connected to the at least one valve (50).

4. A sterilization system comprising:
the sterilization device (10) of claim 1, wherein the at least one B class sterilization cycle program (76b) is in a locked state and cannot be run in a locked state and wherein the sterilization device (10) provides S class sterilization and is adaptable to unlock and provide B class sterilization; and
a code (110) for unlocking the at least one locked B class sterilization cycle.

5. A method of sterilization, the method comprising:
providing a device (10) of claim 1;
placing at least one item in the sterilization chamber (12); and
running at least one S class sterilization cycle (76a) for sterilizing the at least one item.

6. A method of converting a sterilization device (10) for providing S class sterilization into a sterilization device (10) for providing B class sterilization, the method comprising:
providing a device (10) of claim 1, wherein the device (10) is in a first state; and
inputting a code (110) for unlocking the at least one B class sterilization cycle program (76b) for providing the sterilization device (10) with at least one available B class sterilization cycle program (76b) and for converting the sterilization device (10) from a first state wherein the at least one B class sterilization cycle (76b) is locked into a second state wherein the at least one B class sterilization cycle program (76b) is unlocked.

7. A method of sterilization, the method comprising:
providing a device (10) of claim 1, wherein the device (10) is in a first state and the at least one B class sterilization cycle program (76b) is locked;
unlocking the at least one B class sterilization cycle program (76b) to provide at least one available B class sterilization cycle program (76b);
placing at least one item in the sterilization chamber (12); and
running at least one B class sterilization cycle (76b) for sterilizing the at least one item.

8. The sterilization device (10) of claim 1, wherein the plurality of sterilization cycle programs (76) further comprises at least one N class sterilization cycle program; and
wherein in a first state the sterilization device (10) provides sterilization using the at least one N class sterilization cycle program and the at least one B class sterilization cycle program (76b) is locked and unavailable for use by a user.

9. The sterilization system of claim 4
wherein the plurality of sterilization cycle programs (76) of the sterilization device (10) further comprises at least one N class sterilization cycle and
wherein the sterilization device (10) provides N class sterilization and is adaptable to unlock and provide B class sterilization.

10. The sterilization system of claim 9,
wherein the sterilization device comprises a passive steam air removal system and at least one of a steam pulsing system (46) and a vacuum system (48) to provide air removal from the sterilization chamber (12).

## Patentansprüche

1. Sterilisationsvorrichtung (10) umfassend:
eine Sterilisationskammer (12) zum Aufnehmen mindestens eines zu sterilisierenden Gegenstands;
ein Energieversorgungsmittel (100) zur Energieversorgung der Vorrichtung;
ein Mittel zum Bereitstellen eines Sterilisationsmediums (18) zum Sterilisieren des mindestens einen Gegenstands;
wobei das Sterilisationsmedium Dampf für die Dampfsterilisation ist;
und
eine Mehrzahl von Sterilisationszyklusprogrammen (76), umfassend:
mindestens ein Klasse-S-Sterilisationszyklusprogramm (76a); und
mindestens ein Klasse-B-Sterilisationszyklusprogramm (76b);
wobei die Sterilisationsvorrichtung (10) eine Steuereinheit (60) zum Steuern der Sterilisationszyklen der Sterilisationsvorrichtung (10) umfasst
und **dadurch gekennzeichnet ist, dass**
die Sterilisationsvorrichtung (10) in einem ersten Zustand eine Sterilisation unter Verwendung des mindestens einen Klasse-S-Sterilisationszyklusprogramms (76a) bereitstellt und das mindestens eine Klasse-B-Sterilisationszyklusprogramms (76b) gesperrt und für die Verwendung durch einen Benutzer nicht verfügbar ist;
die Sterilisationsvorrichtung (10) in einem zweiten Zustand das mindestens eine B-Klasse-Sterilisationszyklusprogramm entsperrt und für die Verwendung durch einen Benutzer verfügbar bereitstellt;
wobei das mindestens eine gesperrte Klasse-B-Sterilisationszyklusprogramm dazu konfiguriert ist, mit einem Code entsperrt zu werden; und
wobei die Sterilisationsvorrichtung (10) eine Tastatur zum Eingeben des Codes in die Steuereinheit (60) umfasst oder der Code ferngesteuert eingegeben werden kann.

2. Sterilisationsvorrichtung nach Anspruch 1, wobei die Vorrichtung (10) mindestens ein Luftentfernungssystem (40) umfasst und wobei das Luftentfernungssystem (40) dazu konfiguriert ist, Luftentfernung aus der Sterilisationskammer (12) bereitzustellen, und wobei das mindestens eine Luftentfernungssystem (40) ein Dampfpulsierungssystem (46) umfasst, das Wasser, eine Wasserpumpe und einen Wasserheizer umfasst, und wobei die Sterilisationskammer umfasst:
mindestens einen Einlass (28) für mindestens Dampf oder Wasser; und
mindestens einen Auslass (36) für mindestens Luft oder Dampf; und
wobei das Dampfpulsierungssystem mit dem mindestens einen Einlass (28) der Sterilisationskammer (12) verbunden ist.

3. Sterilisationsvorrichtung nach Anspruch 1, wobei die Vorrichtung (10) mindestens ein Luftentfernungssystem (40) umfasst und wobei das Luftentfernungssystem dazu konfiguriert ist, eine Luftentfernung aus der Sterilisationskammer (12) bereitzustellen, und wobei das mindestens eine Luftentfernungssystem (40) ein Vakuumsystem (48) umfasst und wobei die Sterilisationskammer (12) mindestens ein Ventil (50) umfasst und das Vakuumsystem (48) mit dem mindestens ein Ventil (50) verbunden ist.

4. Sterilisationssystem, umfassend:
die Sterilisationsvorrichtung (10) nach Anspruch 1, wobei das mindestens eine Klasse-B-Sterilisationszyklusprogramm (76b) in einem gesperrten Zustand ist und in einem gesperrten Zustand nicht ausgeführt werden kann, und wobei die Sterilisationsvorrichtung (10) eine Klasse-S-Sterilisation bereitstellt und dazu ausgelegt werden kann, Klasse-B-Sterilisation zu entsperren und bereitzustellen; und
einen Code (110) zum Entsperren des mindestens einen gesperrten Klasse-B-Sterilisationszyklus.

5. Verfahren zur Sterilisation, wobei das Verfahren umfasst:
Bereitstellen einer Vorrichtung (10) nach Anspruch 1;
Platzieren mindestens eines Gegenstands in der Sterilisationskammer (12); und
Ausführen mindestens eines Klasse-S-Sterilisationszyklus (76a) zum Sterilisieren des mindestens einen Gegenstands.

6. Verfahren zum Konvertieren einer Sterilisationsvorrichtung (10) zum Bereitstellen von Klasse-S-Sterilisation in eine Sterilisationsvorrichtung (10) zum Bereitstellen von Klasse-B-Sterilisation, wobei das Verfahren umfasst:
Bereitstellen einer Vorrichtung (10) gemäß Anspruch 1, wobei sich die Vorrichtung (10) in einem ersten Zustand befindet; und
Eingeben eines Codes (110) zum Entsperren des mindestens einen Klasse-B-Sterilisationszyklusprogramms (76b) zum Bereitstellen der Sterilisationsvorrichtung (10) mit mindestens einem verfügbaren Klasse-B-Sterilisationszyklusprogramm (76b), und zum Konvertieren der Sterilisationsvorrichtung (10) aus einem ersten Zustand, in dem der mindestens eine Klasse-B-Sterilisationszyklus (76b) gesperrt ist, in einen zweiten Zustand, in dem das mindestens eine Klasse-B-Sterilisationszyklusprogramm (76b) entsperrt ist.

7. Verfahren zur Sterilisation, wobei das Verfahren umfasst:
Bereitstellen einer Vorrichtung (10) nach Anspruch 1, wobei sich die Vorrichtung (10) in einem ersten Zustand befindet und das mindestens eine Klasse-B-Sterilisationszyklusprogramm (76b) gesperrt ist;
Entsperren des mindestens einen Klasse-B-Sterilisationszyklusprogramms (76b), um mindestens ein verfügbares Klasse-B-Sterilisationszyklusprogramm (76b) bereitzustellen;
Platzieren mindestens eines Gegenstands in die Sterilisationskammer (12); und
Ausführen mindestens eines Klasse-B-Sterilisationszyklus (76b) zum Sterilisieren des mindestens einen Gegenstands.

8. Sterilisationsvorrichtung (10) nach Anspruch 1, wobei die Mehrzahl von Sterilisationszyklusprogrammen (76) ferner mindestens ein Klasse-N-Sterilisationszyklusprogramm umfasst; und
wobei die Sterilisationsvorrichtung (10) in einem ersten Zustand eine Sterilisation unter Verwendung des mindestens einen Klasse-N-Sterilisationszyklusprogramms bereitstellt und das mindestens eine Klasse-B-Sterilisationszyklusprogramm (76b) gesperrt und für die Verwendung durch einen Benutzer nicht verfügbar ist.

9. Sterilisationssystem nach Anspruch 4
wobei die Mehrzahl von Sterilisationszyklusprogrammen (76) der Sterilisationsvorrichtung (10) ferner mindestens einen Klasse-N-Sterilisationszyklus umfasst und
wobei die Sterilisationsvorrichtung (10) Klasse-N-Sterilisation bereitstellt und dazu ausgelegt werden kann, Klasse-B-Sterilisation zu entsperren und bereitzustellen.

10. Sterilisationssystem nach Anspruch 9,
wobei die Sterilisationsvorrichtung ein passives Dampf-Luftentfernungssystem und mindestens ein Dampfpulsierungssystem (46) oder ein Vakuumsystem (48) umfasst, um Luftentfernung aus der Sterilisationskammer (12) bereitzustellen.

## Revendications

1. Dispositif de stérilisation (10) comprenant :
une chambre de stérilisation (12) pour loger au moins un article à stériliser ;
un moyen d'alimentation (100) pour alimenter le dispositif ;
un moyen destiné à fournir un milieu de stérilisation (18) pour stériliser l'au moins un article ; le milieu de stérilisation étant de la vapeur pour la stérilisation à la vapeur ;
et
une pluralité de programmes (76) de cycle de stérilisation comprenant :
au moins un programme (76a) de cycle de stérilisation de classe S ; et
au moins un programme (76b) de cycle de stérilisation de classe B ;
le dispositif de stérilisation (10) comprenant un dispositif de commande (60) pour commander les cycles de stérilisation du dispositif de stérilisation (10)
et **caractérisé en ce que**,
dans un premier état, le dispositif de stérilisation (10) fournit une stérilisation à l'aide de l'au moins un programme (76a) de cycle de stérilisation de classe S et l'au moins un programme (76b) de cycle de stérilisation de classe B est verrouillé et non disponible à l'utilisation par un utilisateur ;
dans un second état, le dispositif de stérilisation (10) fournit l'au moins un programme de cycle de stérilisation de classe B déverrouillé et disponible à l'utilisation par un utilisateur ;
dans lequel l'au moins un programme de cycle de stérilisation de classe B verrouillé est configuré pour être déverrouillé à l'aide d'un code ; et
le dispositif de stérilisation (10) comprenant un clavier destiné à saisir en entrée le code dans le dispositif de commande (60) ou le code peut être saisi en entrée à distance.

2. Dispositif de stérilisation selon la revendication 1, le dispositif (10) comprenant au moins un système (40) d'évacuation d'air et dans lequel le système (40) d'évacuation d'air est configuré pour fournir une évacuation de l'air de la chambre de stérilisation (12) et dans lequel l'au moins un système (40) d'évacuation d'air comprend un système (46) à impulsions de vapeur comprenant de l'eau, une pompe à eau et un chauffe-eau et dans lequel la chambre de stérilisation comprend :
au moins une entrée (28) pour au moins l'une parmi la vapeur et l'eau ; et
au moins une sortie (36) pour au moins l'un parmi l'air et la vapeur ; et
dans lequel le système à impulsions de vapeur est relié à l'au moins une entrée (28) de la chambre de stérilisation (12).

3. Dispositif de stérilisation selon la revendication 1, le dispositif (10) comprenant au moins un système (40) d'évacuation d'air et dans lequel le système d'évacuation d'air est configuré pour fournir une évacuation de l'air de la chambre de stérilisation (12) et dans lequel l'au moins un système (40) d'évacuation d'air comprend un système à vide (48) et dans lequel la chambre de stérilisation (12) comprend au moins une soupape (50) et le système à vide (48) est relié à l'au moins une soupape (50).

4. Système de stérilisation comprenant :
le dispositif de stérilisation (10) selon la revendication 1, dans lequel l'au moins un programme (76b) de cycle de stérilisation de classe B est dans un état verrouillé et ne peut pas être exécuté dans un état verrouillé, et le dispositif de stérilisation (10) fournissant une stérilisation de classe S et étant adaptable pour déverrouiller et fournir une stérilisation de classe B ; et
un code (110) destiné à déverrouiller l'au moins un cycle de stérilisation de classe B verrouillé.

5. Procédé de stérilisation, le procédé comprenant :
la fourniture d'un dispositif (10) selon la revendication 1 ;
le placement d'au moins un article dans la chambre de stérilisation (12) ; et
l'exécution d'au moins un cycle (76a) de stérilisation de classe S pour stériliser l'au moins un article.

6. Procédé de conversion d'un dispositif de stérilisation (10) destiné à fournir une stérilisation de classe S en un dispositif de stérilisation (10) destiné à fournir une stérilisation de classe B, le procédé comprenant :
la fourniture d'un dispositif (10) selon la revendication 1, le dispositif (10) étant dans un premier état ; et
la saisie en entrée d'un code (110) pour déverrouiller l'au moins un programme (76b) de cycle de stérilisation de classe B pour fournir au dispositif de stérilisation (10) au moins un programme (76b) de cycle de stérilisation de classe B disponible et pour convertir le dispositif de stérilisation (10) d'un premier état dans lequel l'au moins un programme (76b) de cycle de stérilisation de classe B est verrouillé à un second état dans lequel l'au moins un programme (76b) de cycle de stérilisation de classe B est déverrouillé.

7. Procédé de stérilisation, le procédé comprenant :
la fourniture d'un dispositif (10) selon la revendication 1, le dispositif (10) étant dans un premier état et l'au moins un programme (76b) de cycle de stérilisation de classe B est verrouillé ;
le déverrouillage de l'au moins un programme (76b) de cycle de stérilisation de classe B pour fournir au moins un programme (76b) de cycle de stérilisation de classe B disponible ;
le placement d'au moins un article dans la chambre de stérilisation (12) ; et
l'exécution d'au moins un cycle (76b) de stérilisation de classe B pour stériliser l'au moins un article.

8. Dispositif de stérilisation (10) selon la revendication 1, dans lequel la pluralité de programmes (76) de cycle de stérilisation comprend en outre au moins un programme de cycle de stérilisation de classe N ; et
dans un premier état, le dispositif de stérilisation (10) fournissant une stérilisation à l'aide de l'au moins un programme de cycle de stérilisation de classe N et l'au moins un programme (76b) de cycle de stérilisation de classe B est verrouillé et non disponible à l'utilisation par un utilisateur.

9. Système de stérilisation selon la revendication 4
dans lequel la pluralité de programmes (76) de cycle de stérilisation du dispositif de stérilisation (10) comprend en outre au moins un cycle de stérilisation de classe N et
dans lequel le dispositif de stérilisation (10) fournit une stérilisation de classe N et est adaptable pour déverrouiller et fournir une stérilisation de classe B.

10. Système de stérilisation selon la revendication 9, dans lequel le dispositif de stérilisation comprend un système passif d'évacuation d'air à vapeur et au moins un système d'impulsion de vapeur (46) et un système à vide (48) pour fournir l'évacuation de l'air de la chambre de stérilisation (12).
